Europäisches Patentamt

European Patent Office

Office européen des brevets

(11) Publication number: **0 460 065 B1**

(12) **EUROPEAN PATENT SPECIFICATION**

(45) Date of publication of patent specification: **31.08.94**    (51) Int. Cl.5: **C12N 5/08**, A61K 39/00, A61K 35/14, C12Q 1/70

(21) Application number: **90904107.1**

(22) Date of filing: **08.02.90**

(86) International application number: **PCT/US90/00611**

(87) International publication number: **WO 90/10059 (07.09.90 90/21)**

(54) **A PROCESS FOR THE GENERATION OF PROLIFERATING CD4 LYMPHOCYTES.**

(30) Priority: **21.02.89 US 313421**

(43) Date of publication of application:
**11.12.91 Bulletin 91/50**

(45) Publication of the grant of the patent:
**31.08.94 Bulletin 94/35**

(84) Designated Contracting States:
**AT BE CH DE DK ES FR GB IT LI LU NL SE**

(56) References cited:
**EP-A- 0 247 613**
**EP-A- 0 280 054**
**EP-A- 0 289 896**
**WO-A-88/01642**

**Dialog Information Services, File 154, Medline 83-90, Dialog accession no. 86279678, Thiele DL et al: "Leu-Leu-OMe sensitivity of human activated killer cells: delineation of a distinct class of cytoxic T lymphocytes capable of lysing tumor targets", J Immunol Aug 15 1986, 137 (4) p 1399-1406**

(73) Proprietor: **TERUMO KABUSHIKI KAISHA trading as TERUMO CORPORATION**
**44-1, 2-chome, Hatagaya**
**Shibuya-Ku**
**Tokyo 151 (JP)**

(72) Inventor: **LEUNG, Kam, Hung**
**709 Marshall Road**
**Brookhaven, PA 19015 (US)**
Inventor: **TOWNSEND, Robert, Martin**
**1141 Hollywood Avenue**
**Havertown, PA 19083 (US)**

(74) Representative: **von Kreisler, Alek,**
**Dipl.-Chem. et al**
**Patentanwälte**
**von Kreisler-Selting-Werner**
**Postfach 10 22 41**
**D-50462 Köln (DE)**

Rank Xerox (UK) Business Services
(3.10/3.09/3.3.3)

NATURE, vol. 332, 1988, P.L. Nara et al:
"Quantitative infectivity assay for HIV-1 and
-2", see page 469 - page 470 especially page
470 column 2 line 48 - column 3 line 26

**Description**

Background of the Invention

The helper/inducer subset of T lymphocytes, which are important immunoregulatory cells involved in helper/inducer immune functions, express the CD4 surface antigen on their cell surface. CD4 is a 55 kDa cell surface protein which has been implicated in the recognition of MHC class II antigens. (Biddison et al. (1982) J. Exp. Med. 156: 1065-1076). Such CD4-positive (CD4+) T-cells comprise approximately 45% of human peripheral blood lymphocytes (PBL) (Ledbetter et al. (1981) J. Exp. Med. 153:310). The CD4 antigen is also found in low densities on monocytes (Wood et al. (1982) J. Immunol. 131:212). Currently, the most common methods of purifying CD4+ lymphocytes from peripheral blood involves the use of monoclonal antibodies for the affinity removal or specific depletion of cell types. These affinity separation methods include panning (Engleman et al. (1981) J. Immunol. 127:2124), cell sorting (Parks and Herzenberg (1984) Methods Enzymol. 108:197-241), and complement killing (McCluskey et al. (1984) Int. J. Cell Cloning 2:296-303).

The panning method of cell separation involves a petri dish which is coated with antibodies specific for a cell surface marker present on the subpopulation(s) of lymphocytes or cells to be isolated. Cells which specifically adhere to the dish are separated from those cells which do not adhere. This is one of the better methods for isolating CD4+ lymphocytes, but this method is limited by the inefficiency in isolating pure populations and the difficulty in adapting to large scale. Further, panning is a cumbersome technique and is costly due to the use of antibodies.

For the cell sorting method of cell separation, cells are fluorescent-labeled, usually with particular fluorescent-labeled antibody, and the cells are selected by the cell sorter based on the presence or absence of the antibody. This process is expensive and excessively time consuming since sorting can only be done on a small scale. It is also difficult to maintain sterility during the sorting.

In the complement killing method of cell isolation antibodies specific for the undesirable population of cells are added to the cell mixture and complement is then added to specifically lyse the antibody-bound cells. This method is very inefficient at removing all of the unwanted cells and it is impractical on larger scales. Other methods of cell separation exist, such as those including the use of magnetic beads or affinity columns (Braun et al. (1982) J. Immunol. Methods 54:251-258) and have many of the same limitations as the above methods.

Further, the treatment of human peripheral lymphocytes with L-leucine methyl ester to kill lysosome containing cells is known from WO 88/01642.

Summary of the Invention

The present invention relates to a method of generating and proliferating a lymphocyte population containing at least 90% CD4+ lymphocytes which comprises:

(a) treating peripheral blood mononuclear cells with a 5-10 mM of L-leucyl-leucine methyl ester or 5-10 mM of L-leucine methyl ester to deplete monocytes and large granular lymphocytes and then, without positive selection or affinity removal of non-CD4+lymphocytes,

(b) continually culturing the peripheral blood mononuclear cells in culture media containing T-cell stimulant or interleukin-2 or both at 37°C, for 4-21 days.

The present method eliminates the necessity for cumbersome positive selection and affinity separation processes. This method is most practical due to its efficiency in expanding CD4 T-cells, its ability to be adapted to large scale, its technical simplicity, and its cost effectiveness.

When attempts are made to directly expand in cell number lymphocytes from PBMC, the phenotype of the resulting cells tends to vary greatly, but generally such expanded populations of PBMCs contain low amounts of CD4+ cells and high amounts of CD8+ or Leu 19 cells. We have discovered that when PBMC are first treated with L-leucine methyl ester (LME) or L-leucyl-leucine methyl ester (LLME) and subsequently stimulated with mitogens, CD4-positive cells are found to dominate the cultures as they expand. Such cultures are shown to expand in cell number 10 to 1000-fold depending on the type of stimuli utilized and maintain a purity of greater than 90% CD4-positive lymphocytes.

Certain amino acid methyl esters are readily absorbed by the lysosomes of manocytes where they are hydrolyzed to the corresponding amino acid and methanol (Theile et al. (1983) J. Immunol. 131:2282-2290). The free amino acid accumulates in the lysosomes, creating an osmotic imbalance. Water consequently fills the lysosomes causing them to swell and burst and thus kills the cell. In the case of LME, the free amino acid and any residual LME is converted to leucyl-leucine methyl ester which is released upon destruction of the monocytes (Theile et al. (1985) J. Immunol. 134:786-793).

The invention further relates to the use of a lymphocyte population obtained by the method of claims 1 to 4 for the production of a medicament for treatment of a disease characterized by a deficiency in CD4+ lymphocytes, whereby peripheral blood mononuclear cells are obtained from the patient and treated by the process of claim 1 to provide a CD4+ lymphocyte-enriched cell population, and the CD4+ lymphocyte-enriched cell population is returned to the patient;

and a composition comprising an aqueous suspension of human lymphocytes obtained by the methods of claims 1-4, at least 90% of which are CD4+ lymphocytes, and interleukin-2;

a method of generating and proliferating a tumor infiltrating lymphocyte population enriched for CD4+ lymphocytes which comprises:

(a) treating tumor infiltrating lymphocytes with L-leucyl-leucine methyl ester to deplete monocytes and large granular lymphocytes and then, without positive selection or affinity removal of non-CD4+ lymphocytes,

(b) continually culturing the tumor infiltrating lymphocytes in culture media containing T-cell stimulant and interleukin-2 at 37°C;

a method of producing a human immunodeficiency virus wherein peripheral blood mononuclear cells are obtained from a patient infected with the virus and treated by the process of claim 1 to provide a CD4+ lymphocyte-enriched cell population containing virus-infected cells, then the virus is isolated from the cell population;

a method for detection of a human immunodeficiency virus in peripheral blood mononuclear cells which comprises treating the cells by the process of claim 1 to provide a CD4+ lymphocyte-enriched cell population containing virus-infected cells, if present, then assaying the cells for presence of the virus;

a method for producing autologous target cells for use in assays to determine presence of cytolytic effector cells in a person infected with a human immunodeficiency virus which comprises obtaining peripheral blood mononuclear cells from the person, treating the cells by the method of claim 1, then adding envelope exterior glycoprotein of the virus to produce the target cells.

The LLME may be subsequently absorbed by cytotoxic lymphocytes which are not affected directly by LME and destroys them in a similar fashion. LLME directly added to the cells behaves in a similar manner (Lipsky and Theile, U. S. Patent 4,752,602; Theile and Lipsky (1986) J. Immunol. 136:1038-1048).

When tumor infiltrating lymphocytes (TIL) cells are expanded from tumor cell preparations, CD8-bearing cells tend to dominate the cultures (Topalian et al. (1987) J. Immunol. Methods 102:127-141). By employing this new method of treating the tumor cell with LME or LLME prior to culture, the resultant expanded cell cultures are found to be greatly enriched for CD4-bearing lymphocytes.

The use of LME or LLME on tumor cell preparations in a process to generate a cell population highly enriched for CD4-positive lymphocytes has not been previously reported. The purification of CD4 T-cells from peripheral blood, aided by the use of these compounds, has previously been described (Geppert and Lipsky (1987) J. Immunol. 138:1660-1666). However, the procedure of Geppert and Lipsky differs from the present process in several respects. First, Geppert and Lipsky use an elaborate, multistep purification process involving several affinity depletion and positive selection steps to purify the CD4+ lymphocytes, whereas we do not. For this purification, 6 purification steps are used In addition to the LME treatment step, including two panning steps. These steps are time consuming, difficult to perform, and impractical on larger scales. Lipsky et al. have also used the same basic strategy to isolate CD8 T-cells (Theile and Lipsky (1988) Clinical Immunology and Immunopathology 48:405-423). Secondly, Geppert and Lipsky do not expand these cells for use in immunotherapies; rather, they are merely studying the various parameters necessary for the activation of purified T-cell populations.

Detailed Description of the Invention

PBMC are typically Isolated from the whole blood product by means of Ficoll-Hypaque (Boyun (1989) Tissue Antigens 4:269-274).

Some of the human lymphoid subsets in PMBC preparations, as defined by surface antigens, are shown in Table 1.

## TABLE 1

## HUMAN LYMPHOID SUBSETS
## DEFINED BY SURFACE ANTIGENS

| CD19 (B-CELLS) | CD2 (ALL T AND NK CELLS) | | | CD14 (MONOCYTES) |
|---|---|---|---|---|
| | CD3 (T-CELLS) | | | |
| | CD4 (HELPER) | CD8 (CYT/SUPP) | CD16 (LGL/NK) | |
| | | | LEU 19 (LGL/NK) | |

The nomonclature commonly used for lymphocyte subset are given in Table 2.

TABLE 2

| Lymphocyte Subsets and Specific Monoclonal Antibodies (Mabs) (B-D = Bectin Dickinson) | | | | |
|---|---|---|---|---|
| CD# Other names | CD2 T11 | CD3 T3 | CD4 T4 | CD8 T8 |
| MAbs: | | | | |
| B-D Ortho | Leu-5 OKT-11 | Leu-4 OKT-3 | Leu-3 OKT-4 | Leu-2 OKT-8 |

The isolated PBMC are then incubated with a concentration of LME or LLME for sufficient time, to effectively reduce or eliminate cytotoxic CD8 T-cells, NK cells and LAK precursors.

## TABLE 3

The effect of LME concentration and length of treatment OF PBMC on CD4 generation. PBMC were isolated from a Red cross Buffycoat by Ficoll separation. Cells were set at $1 \times 10^7$/mL in AIM V media and partitioned into fifths. Each fifth was treated with a different concentration of LME (0, 0.5, 1.0, 5.0, and 10 mM). At several time points (30, 45, 60, and 75 min.) one fourth of the cells were removed from each and washed 2 times in AIM V. Cells were then cultured at $1 \times 10^6$/mL in AIM V with 400 U/mL IL2 added. PHA was added to each culture at 1 μg/mL and cultured for 6 days. FACS analysis was performed on day 6. Data are expressed as % $CD4^+$.

| | Duration of Treatment (min) | | | |
|---|---|---|---|---|
| LME [mM] | 30 | 45 | 60 | 75 |
| 0 | 35 | 35 | 35 | 35 |
| 0.5 | 52 | 53 | 53 | 57 |
| 1.0 | 60 | 56 | 58 | 59 |
| 5.0 | 69 | 93 | 98 | 98 |
| 10 | 70 | 95 | 98 | 99 |

As shown in Table 3, treatment of PBMCs for 45 to 75 minutes using >1.0 to 10 mM LME is effective in generating cultures of content of >90% CD4 cells. After treatment for approximately 60 min, cells are washed 2-times to remove the LME or LLME. Cells are then cultured in AIM V cell culture medium at 37°C with the desired stimuli, for example, lectin (such as phytohemaglutin [PHA]) or antigen (for example, a tumor antigen), with or without the addition of exogenous IL2. The IL2 activity units are expressed throughout as BRMP (Biological Response Modifier Program) units (U). In 4 to 7 days, cell growth begins and continues for 2 to 3 weeks in the presence of IL2. The resultant population of cells is composed of greater than 90% $CD4^+$ cells following a cell expansion 10 to 1000-fold.

Tables 4 and 5 summarize typical results using the process of the invention. Briefly, PBMC were treated with 5 mM LME (Sigma) for 60 minutes and continuously cultured in 400 U/mL IL2 and AIM V media (Gibco). Cultures were stimulated with either PHA (Wellcome) at 1 μg/mL, irradiated IM9 (ATCC) tumor cells at a responder cell to stimulator cell ratio (R:S) of 5:1, or nothing.

## TABLE 4

PBMC were incubated for 60 min. in the presence or absence of 5 mM LME. Cells were washed 2 times to remove the LME. Cells were cultured in AIM V media serum free at a density of $1 \times 10^6$ cells/mL and 400 U/mL IL2. Mitogens were added to the indicated cultures (PHA=1 $\mu$g/mL and IM9=20%). Cells were allowed to grow and counted at various time points via trypan blue exclusion. Total expansion was calculated from these cell counts and expressed as fold expansion. On the days cell counts were done, fresh media and IL2 were given to the cultures.

### Cumulative Proliferation

|  | Day 0 | Day 7 | Day 10 | Day 13 | Day 18 | Day 21 |
|---|---|---|---|---|---|---|
| **Untreated** | | | | | | |
| IL2 | 1.00 | 1.94 | 0.81 | 4.99 | 10.67 | 4.27 |
| IL2+ PHA | 1.00 | 5.18 | 14.81 | 54.52 | 303.12 | 351.62 |
| IL2+ IM9 | 1.00 | 2.80 | 6.10 | 21.36 | 54.69 | 16.41 |
| **LME Treated** | | | | | | |
| 1L2 | 1.00 | 0.56 | 1.68 | 3.02 | 10.64 | 6.39 |
| IL2+ PHA | 1.00 | 2.68 | 8.31 | 23.93 | 95.71 | 160.79 |
| IL2+ IM9 | 1.00 | 1.74 | 4.70 | 13.72 | 59.26 | 67.56 |

## TABLE 5

The cultures in Table 4 were periodically tested for % CD4$^+$ lymphocytes present. Leu 3a (Becton Dickinson) was used to define CD4 and a FACSCAN (Becton Dickinson) was used for the analysis. Data is expressed as % CD4$^+$.

|  | Day 0 | Day 13 | Day 21 |
|---|---|---|---|
| **Untreated** |  |  |  |
| IL2 | 44 | 41 | ND |
| IL2+ PHA | 44 | 31 | ND |
| IL2+ IM9 | 44 | 2 | ND |
| **LME Treated** |  |  |  |
| IL2 | 57 | 95 | 92 |
| IL2+ PHA | 57 | 97 | 92 |
| IL2+ IM9 | 57 | 98 | 96 |

Cell growth (Table 4) was similar for both the treated and untreated cells. The phenotype of the untreated cells on day 15 varied greatly with the content of CD4-positive cells very low (all less than 50%). In contrast, the LME-treated cell cultures all exhibit a cell content of greater than 90% CD4-positive cells on day 15 and maintain this high level of CD4-positive cell content throughout culture to day 21.

Experiments were also carried out utilizing LLME at 0.5 mM in place of LME. Tables 6 and 7 illustrate similar findings to the results obtained using LME.

## TABLE 6

PBMC were incubated for 60 min. in the presence or absence of 500 $\mu$M LLME. Cells were washed 2 times to remove the LLME. Cells were cultured in AIM V media (serum free) at a density of $1 \times 10^6$ cells/mL and 400 U/mL IL2. Mitogens were added to the indicated cultures (PHA=1 $\mu$g/mL and IM9=20%). Cells were allowed to grow and counted at various time points via trypan blue exclusion. Total expansion was calculated from these cell counts and expressed as fold expansion. On the days cell counts were done, fresh medium and IL2 were given to the cultures. A seventh culture was set up without any IL2. This culture was stimulated with PHA (1 $\mu$g/mL) and the growth and CD4 purity (Table 7) was compared to the culture with the IL2 and stimulated with PHA.

| Proliferation Overall | | | | | | | | |
|---|---|---|---|---|---|---|---|---|
| Day: | 0 | 4 | 6 | 8 | 11 | 14 | 18 | 21 |
| Untreated | | | | | | | | |
| IL2 | 1.00 | 1.00 | 1.04 | 1.70 | 5.95 | 3.93 | 6.07 | 5.24 |
| IL2+ IM9 | 1.00 | 1.00 | 1.24 | 2.53 | 6.07 | 9.71 | 38.85 | 134.44 |
| IL2+ PHA | 1.00 | 2.00 | 4.12 | 14.09 | 72.7 | 147.99 | 395.52 | 1447.61 |
| LLME Treated | | | | | | | | |
| IL2 | 1.00 | 1.00 | 0.32 | 0.28 | 1.78 | 3.28 | 9.89 | 10.09 |
| IL2+ IM9 | 1.00 | 1.00 | 0.72 | 1.82 | 4.77 | 9.16 | 41.93 | 132.50 |
| IL2+ PHA | 1.00 | 1.00 | 1.41 | 4.06 | 14.29 | 39.45 | 198.05 | 938.74 |
| PHA | 1.00 | 1.00 | 1.08 | 2.32 | 8.07 | 10.98 | 37.11 | 29.69 |

## TABLE 7

The cultures in Table 6 were periodically tested for % CD4$^+$ lymphocytes present. Leu 3a (Becton Dickinson) was used to define CD4 and a FACSCAN (Becton Dickinson) was used for the analysis. Data is expressed as % CD4$^+$

|  | Day 0 | Day 4 | Day 8 | Day 14 | Day 21 |
|---|---|---|---|---|---|
| Untreated |  |  |  |  |  |
| IL2 | 48 | 59 | 47 | 36 | 41 |
| IL2+ IM9 | 48 | 48 | 38 | 27 | 18 |
| IL2+ PH | 48 | 67 | 63 | 50 | 37 |
| LLME Treated |  |  |  |  |  |
| IL2 | 56 | 91 | 91 | 93 | 95 |
| IL2+ IM9 | 56 | 83 | 92 | 95 | 95 |
| IL2+ PHA | 56 | 94 | 90 | 87 | 72 |
| PHA | 56 | 93 | 92 | 91 | 88 |

Additionally, one culture received PHA, but never any exogenous IL2. Rapid growth was seen initially; however, this growth tapered off after 2 weeks in culture (Table 6). The level of CD4 cells was somewhat higher in the culture which did not receive IL2 than the culture containing PHA plus IL2 (Table 7). Thus, the addition of IL2 appeared to promote CD8-cell growth to a greater extent than CD4-cell growth.

Table 8 shows a summary of the phenotypes of PBMCs for 8 different donors following treatment with LME and cultured in IL2, without prior mitogen treatment (IL2) or with prior mitogen treatment (IM9 or PHA), as described above. The data show the percent of CD4 and CD8 cells in the cultures at day 0 and after culture for approximately two weeks. Generally the percent of CD4 cells for cultures treated with LME is greater than 90% (22 out of 24 cultures).

10

| TABLE 8 | | | | | | |
|---|---|---|---|---|---|---|
| DONOR | | Day 0 | | | Day 13-16 | |
| | | CD4 | CD8 | | CD4 | CD8 |
| 1 | | | | | | |
| | Control | 55 | 16 | IL2 | ND | ND |
| | | | | IM9 | ND | ND |
| | | | | PHA | ND | ND |
| | LME | 65 | 10 | IL2 | ND | ND |
| | | | | IM9 | 99 | 2 |
| | | | | PHA | ND | ND |
| 2 | | | | | | |
| | Control | 42 | 16 | IL2 | 49 | 46 |
| | | | | IM9 | 11 | 67 |
| | | | | PHA | 43 | 63 |
| | LME | 27 | 7 | IL2 | 91 | 7 |
| | | | | IM9 | 90 | 11 |
| | | | | PHA | 83 | 20 |
| 3 | | | | | | |
| | Control | 44 | 20 | IL2 | 41 | 37 |
| | | | | IM9 | 2 | 39 |
| | | | | PHA | 31 | 75 |
| | LME | 57 | 17 | IL2 | 95 | 3 |
| | | | | IM9 | 98 | 2 |
| | | | | PHA | 97 | 3 |
| 4 | | | | | | |
| | Control | 54 | 13 | IL2 | ND | ND |
| | | | | IM9 | ND | ND |
| | | | | PHA | ND | ND |
| | LME | 68 | 6 | IL2 | 96 | 2 |
| | | | | IM9 | 99 | 1 |
| | | | | PHA | 99 | 0 |

| TABLE 8 (continued) | | | | | | |
|---|---|---|---|---|---|---|
| **5** | | | | | | |
| | Control | 48 | 19 | IL2 | ND | ND |
| | | | | IM9 | ND | ND |
| | | | | PHA | ND | ND |
| | LME | 58 | 13 | IL2 | 92 | 7 |
| | | | | IM9 | 96 | 3 |
| | | | | PHA | 88 | 14 |
| **6** | | | | | | |
| | Control | 45 | 22 | IL2 | ND | ND |
| | | | | IM9 | ND | ND |
| | | | | PHA | ND | ND |
| | LME | 72 | 10 | IL2 | 95 | 3 |
| | | | | IM9 | 98 | 1 |
| | | | | PHA | 98 | 2 |
| **7** | | | | | | |
| | Control | ND | ND | IL2 | ND | ND |
| | | | | IM9 | ND | ND |
| | | | | PHA | ND | ND |
| | LME | 60 | 12 | IL2 | 95 | 2 |
| | | | | IM9 | 96 | 2 |
| | | | | PHA | ND | ND |
| **8** | | | | | | |
| | Control | 54 | 13 | IL2 | ND | ND |
| | | | | IM9 | ND | ND |
| | | | | PHA | ND | ND |
| | LME | 68 | 6 | IL2 | 96 | 3 |
| | | | | IM9 | 99 | 1 |
| Summary | | | | | | |
| | Control | 49 | 17 | IL2 | 45 | 42 |
| | STD | 5 | 3 | STD | 4 | 5 |
| | | | | IM9 | 7 | 53 |
| | | | | STD | 5 | 14 |
| | | | | PHA | 37 | 69 |
| | | | | STD | 6 | 6 |

|  | TABLE 8 (continued) | | | | | |
|---|---|---|---|---|---|---|
| LME | 59 | 10 | IL2 | 94 | 4 |
| STD | 13 | 4 | STD | 2 | 2 |
|  |  |  | IM9 | 97 | 3 |
|  |  |  | STD | 3 | 3 |
|  |  |  | PHA | 94 | 7 |
|  |  |  | STD | 6 | 8 |

ND = not done

The method of obtaining cultures of cells highly enriched for CD4-positive lymphocytes was applied to tumor preparations in an attempt to obtain the expansion of CD4[+] tumor infiltrating lymphocytes (TIL) (Topalian et al. (1988) J. Clinical Oncology 6:839-853). Individual frozen tumor preparations were thawed and aliquoted into 4 samples. Samples received either 5 mM LME treatment, 0.5 mM LLME treatment, 5 mM phenylalanine methyl ester (PME) treatment, or no treatment. These treatments were carried out for approximately 1 hour and the cultures were then washed twice with AIM V medium. Cells were resuspended in AIM V media with 4000 U/mL of IL2, at a concentration of $5 \times 10^6$ cells/ml and cultured at 37°C until expansion of cells ceased. On days 21 and 27 fluorescent-activated cell sort (FACS) analysis was performed to determine the phenotypes of the different cultures. Table 9 summarizes the results of this analysis and Table 10 illustrates the expansion of each of the TIL cultures. On both days 21 and 27, the relative CD4-cell content of the LLME culture is enhanced relative to the culture which was not treated with LLME. Proliferation for the LLME-treated and untreated cultures was comparable. It is noted that in the experiment of Table 9, LME was ineffective at enriching CD4[+] cell content. In general, the effectiveness of LME will depend on the level of sufficient monocytes in the culture which are necessary to convert LME to LLME.

## TABLE 9

Tumor cells were received from the NCI and prepared for culture by the standard protocol. Samples were frozen and stored in liquid nitrogen for later use. These samples were thawed out and *washed 2 times. Cells were set at $1x10^7/mL$ in AIM V* medium. Four aliquots were taken and treated with either PME (5 mM), LME (5 mM), LLME (500 $\mu$M) or nothing for 60 min. After treatment, cells were washed 2 times in AIM V media and cultured at $5x10^5$ cells/mL in AIM V media with 4000 U/mL IL2. Cells were allowed to grow and the phenotypes of the cultures were compared on days 0, 21, and 27 via FACS analysis. Data is expressed as % $CD4^+$.

| Culture | Day 0 | Day 21 | Day 27 |
|---------|-------|--------|--------|
| Control | 23 | 11 | 5 |
| PME | 24 | 11 | 8 |
| LME | 22 | 4 | 2 |
| LLME | 23 | 40 | 48 |

## TABLE 10

The tumor cells received from the NCI and treated with different amino acid methyl esters were cultured in AIM V media with 4000 U/mL IL2, as described in Table 9. Cultures were allowed to grow and expansion was tracked by cell counting.

| | Total Cell Number $(x10^6)$ | | | | |
|---|---|---|---|---|---|
| Culture | Day 0 | Day 9 | Day 16 | Day 19 | Day 33 |
| Control | 7.0 | 2.9 | 22.8 | 20.2 | 197.0 |
| PME | 7.0 | 4.4 | 26.7 | 31.2 | 107.0 |
| LME | 4.5 | 4.3 | 12.8 | 12.0 | 31.5 |
| LLME | 5.5 | 1.9 | 13.0 | 30.2 | 720.0 |

The present method provides for the efficient generation of large numbers of CD4 lymphocytes. This method has both therapeutic and analytical applications. Therapeutically, CD4 cells can be used in the treatment of disease states such as cancer or diseases associated with CD4 cell deficiency or disfunction, such as AIDS. In cancer, CD4 cells derived from TIL preparations can be used as immunomodulators to specifically attack tumors by providing the necessary helper functions in vivo to reduce tumors (Rosenstein et al. (1984) J. Immunol. 132:2117-2122). Also CD4 cells derived from the peripheral blood could be used in a similar fashion to provide the necessary helper immune function to effect tumor reduction.

The TIL-derived CD4[+] lymphocytes can be used alone or in combination with other cell types or effector cell populations. CD4 TIL cells can be administered in vivo along with effector cells derived from standard TIL preparations or effector cells derived from the peripheral blood. CD4 cells derived from the peripheral blood could also be administered with the TIL-derived CD4+ cells in an attempt to immunomodulate a cancer patient. CD4 cells derived from either TIL or PMBCs could be added in vitro to cultures or autologous effector cells to possibly enhance the growth or functions of these effectors.

In disease states such as AIDS, large numbers of CD4-positive lymphocytes could be derived from patient's peripheral blood for reinfusion to reconstitute the depleted CD4 population in the patient's blood. In order to do this, PBL from patients must be treated with the proper concentration of an antiviral agent, such as AZT, to inhibit the replication of the virus in vitro (Perno et al. (1988) J. Exp. Med. 168:1111-1125). Also soluble recombinant CD4 (r-CD4) could be used to prevent the infection of new cells in vitro (Fisher et al. (1988) Nature 331:76-78). One could possibly use this soluble r-CD4 conjugated to a toxin to eliminate infected cells prior to culturing (Till et al. (1988) Science 424:1166-1168).

We tested whether the presence of AZT would interfere with the ability to generate CD4 cell using the process of the invention. Table 11, shows the result of adding AZT to cultures of PBMC which were treated with 5 mM LME, stimulated with IM9 tumor cells, and subsequently cultured in AIM V medium with 400 U/mL r-IL2. AZT was added to the cultures at varying concentration and maintained throughout the duration of the culture. AZT was found to inhibit the growth of these cells to some extent; however, growth is still significant (100-fold expansion) at effective concentrations of AZT (0.1 $\mu$g/ml). As shown in Table 12 AZT did not reduce the relative level of CD4 cell content in these cultures.

## TABLE 11

PBMC were treated with 5 mM LME for 60 min. Cells were cultured in AIM V media with 400 U/ml IL2 at a density of $1x10^6$ cells/mL. Cultures were stimulated with 20% IM9 to induce expansion. Cultures were given varying amounts of AZT (1, 0.1, 0.01 and 0 $\mu$g/mL). Cells were allowed to grow and expansion was tracked and expressed as fold expansion.

### Cumulative Proliferation

| Day: | 0 | 8 | 11 | 13 | 15 | 18 | 21 |
|------|------|------|------|------|-------|-------|--------|
| AZT ($\mu$g/mL) | | | | | | | |
| 0 | 1.00 | 1.00 | 2.96 | 8.58 | 20.60 | 97.24 | 260.60 |
| .01 | 1.00 | 1.00 | 2.40 | 5.52 | 15.79 | 60.94 | 146.25 |
| .1 | 1.00 | 1.00 | 2.00 | 5.00 | 16.60 | 44.82 | 107.57 |
| 1 | 1.00 | 1.00 | 1.74 | 4.87 | 11.11 | 37.77 | 67.98 |

TABLE 12

The cultures in Table 11 were tested for % CD4$^+$ and % CD8$^+$ lymphocytes present on Day 15. Leu 3a (Becton Dickinson) was used to define CD4, Leu 2b (Becton Dickinson) was used to define CD8 and a FACSCAN (Becton Dickinson) was used for the analysis. Data is expressed as % Leu 3+ and % Leu 2+.

|  | Leu 3 | Leu 2 |
|---|---|---|
| MLT Control | 99 | 1 |
| AZT 1 $\mu$G/mL | 99 | 0 |
| AZT 0.1 $\mu$G/mL | 100 | 0 |
| AZT 0.01 $\mu$G/mL | 98 | 1 |

Cells grown from an HIV patient in the above fashion could be reinfused into the patient or used as an in vitro diagnostic tool to test cellular responses against HIV determinants. By loading these cells with the HIV envelope protein gp120, they could be utilized as either targets in cytotoxicity assays (Lyerly et al. (1987) Proc. Natl. Acad. Sci. USA 4:4601-4605) or stimulators to study specific responses to gp120.

Other disease states for which this method could provide possible therapeutic benefit would include multiple sclerosis (Chofflon et al. (1988) Annals of Neurology 24:185-191), chronic synovitis (Kingsley et al. (1988) Scand. J. of Immunol. 28:225-232), systemic lupus erythematosus (Raziuddin et al. (1988) Clin. and Exper. Imnunol. 72:446-449) and any other disease states in which a CD4 deficiency is implicated.

The ability to generate large numbers of CD4$^+$ lymphocytes could be beneficial for in vitro antibody production. CD4$^+$ T-Cells could be generated by the present method and added to cultures of human splenocytes immunized in vitro against antigens in an attempt to produce antibodies (Ho, Du Pont patent application U. S. Patent application 736,660). This technology could aid in the development of the ability to produce large numbers of human antibodies in vitro.

**Claims**
**Claims for the following Contracting States : AT, BE, CH, DE, DK, FR, GB, IT, LI, LU, NL, SE**

1.  A method of generating and proliferating a lymphocyte population containing at least 90% CD4$^+$ lymphocytes which comprises:
    (a) treating peripheral blood mononuclear cells with 5-10 mM of L-leucyl-leucine methyl ester or 5-10 mM of L-leucine methyl ester to deplete monocytes and large granular lymphocytes and then, without positive selection or affinity removal of non-CD4$^+$ lymphocytes,
    (b) continually culturing the peripheral blood mononuclear cells in culture media containing T-cell stimulant or interleukin-2 or both at 37 ° C, for 4-21 days.

2.  Method of claim 1 wherein, in step (b), the cells are cultured in media containing interleukin-2 in addition to T-cell stimulant.

3. Method of claim 1 or 2 wherein the CD4$^+$ cells are proliferated by about 10 to 1000 fold.

4. Method of claim 2 wherein the CD4$^+$ cells are proliferated by at least 50 fold.

5. Use of a lymphocyte population obtained by the method of claims 1 to 4 for the production of a medicament for treatment of a disease characterized by a deficiency in CD4$^+$ lymphocytes.

6. Use according to claim 5 whereby the cells obtained from virus infected patients are cultured in step (b) In the presence of an antiviral agent in a concentration effective to inhibit viral replication and/or binding.

7. Use according to claim 6 wherein the patient is infected with a human immunodeficiency virus.

8. Use according to claim 7 wherein the cells are cultured in step (b) in the presence of azidothymidine.

9. Use according to claim 7 wherein the cells are cultured in step (b) in the presence of soluble recombinant CD4.

10. A method of generating and proliferating a tumor infiltrating lymphocyte population enriched for CD4$^+$ lymphocytes which comprises:
    (a) treating tumor infiltrating lymphocytes with L-leucyl-leucine methyl ester to deplete monocytes and large granular lymphocytes and then, without positive selection or affinity removal of non-CD4$^+$ lymphocytes,
    (b) continually culturing the tumor infiltrating lymphocytes in culture media containing T-cell stimulant and interleukin-2 at 37 °C.

11. Method of claim 10 wherein the resulting tumor infiltrating lymphocyte population contains at least 40% CD4$^+$ lymphocytes.

12. Use of the lymphocyte population obtained by the method of claim 11 for the production of a medicament for treating cancer.

13. A composition comprising an aqueous suspension of human lymphocytes obtained by the methods of claims 1-4, at least 90% of which are CD4$^+$ lymphocytes, and interleukin-2.

14. Composition of claim 13 wherein the lymphocytes are peripheral blood lymphocytes.

15. Method of producing a human immunodeficiency virus wherein peripheral blood mononuclear cells are obtained from a patient infected with the virus and treated by the process of claim 1 to provide a CD4$^+$ lymphocyte-enriched cell population containing virus-infected cells, then the virus is isolated from the cell population.

16. Method for detection of a human immunodeficiency virus in peripheral blood mononuclear cells which comprises treating the cells by the process of claim 1 to provide a CD4$^+$ lymphocyte-enriched cell population containing virus-infected cells, if present, then assaying the cells for presence of the virus.

17. Method of claim 16 wherein the cells are assayed for presence of the virus by hybridization assay or immunoassay.

18. Method for producing autologous target cells for use in assays to determine presence of cytolytic effector cells in a person infected with a human immunodeficiency virus which comprises obtaining peripheral blood mononuclear cells from the person, treating the cells by the method of claim 1, then adding envelope exterior glycoprotein of the virus to produce the target cells.

**Claims for the following Contracting State : ES**

1. A method of generating and proliferating a lymphocyte population containing at least 90% CD4$^+$ lymphocytes which comprises:

(a) treating peripheral blood mononuclear cells with 5-10 mM of L-leucyl-leucine methyl ester or 5-10 mM of L-leucine methyl ester to deplete monocytes and large granular lymphocytes and then, without positive selection or affinity removal of non-CD4$^+$ lymphocytes,

(b) continually culturing the peripheral blood mononuclear cells in culture media containing T-cell stimulant or interleukin-2 or both at 37°C, for 4-21 days.

2. Method of claim 1 wherein, in step (b), the cells are cultured in media containing interleukin-2 in addition to T-cell stimulant.

3. Method of claim 1 or 2 wherein the CD4$^+$ cells are proliferated by about 10 to 1000 fold.

4. Method of claim 2 wherein the CD4$^+$ cells are proliferated by at least 50 fold.

5. Use of a lymphocyte population obtained by the method of claims 1 to 4 for the production of a medicament for treatment of a disease characterized by a deficiency in CD4$^+$ lymphocytes.

6. Use according to claim 5 whereby the cells obtained from virus infected patients are cultured in step (b) in the presence of an antiviral agent in a concentration effective to inhibit viral replication and/or binding.

7. Use according to claim 6 wherein the patient is infected with a human immunodeficiency virus.

8. Use according to claim 7 wherein the cells are cultured in step (b) in the presence of azidothymidine.

9. Use according to claim 7 wherein the cells are cultured in step (b) in the presence of soluble recombinant CD4.

10. A method of generating and proliferating a tumor infiltrating lymphocyte population enriched for CD4$^+$ lymphocytes which comprises:

(a) treating tumor infiltrating lymphocytes with L-leucyl-leucine methyl ester to deplete monocytes and large granular lymphocytes and then, without positive selection or affinity removal of non-CD4$^+$ lymphocytes,

(b) continually culturing the tumor infiltrating lymphocytes in culture media containing T-cell stimulant and interleukin-2 at 37°C.

11. Method of claim 10 wherein the resulting tumor infiltrating lymphocyte population contains at least 40% CD4$^+$ lymphocytes.

12. Use of the lymphocyte population obtained by the method of claim 11 for the production of a medicament for treating cancer.

13. A process for preparing a composition comprising an aqueous suspension of human lymphocytes obtained by the methods of claims 1-4, at least 90% of which are CD4$^+$ lymphocytes, and interleukin-2.

14. Process of claim 13 wherein the lymphocytes are peripheral blood lymphocytes.

15. Method of producing a human immunodeficiency virus wherein peripheral blood mononuclear cells are obtained from a patient infected with the virus and treated by the process of claim 1 to provide a CD4$^+$ lymphocyte-enriched cell population containing virus-infected cells, then the virus is isolated from the cell population.

16. Method for detection of a human immunodeficiency virus in peripheral blood mononuclear cells which comprises treating the cells by the process of claim 1 to provide a CD4$^+$ lymphocyte-enriched cell population containing virus-infected cells, if present, then assaying the cells for presence of the virus.

17. Method of claim 16 wherein the cells are assayed for presence of the virus by hybridization assay or immunoassay.

**18.** Method for producing autologous target cells for use in assays to determine presence of cytolytic effector cells in a person infected with a human immunodeficiency virus which comprises obtaining peripheral blood mononuclear cells from the person, treating the cells by the method of claim 1, then adding envelope exterior glycoprotein of the virus to produce the target cells.

**Patentansprüche**
**Patentansprüche für folgende Vertragsstaaten : AT, BE, CH, DE, DK, FR, GB, IT, LI, LU, NL, SE**

**1.** Verfahren zum Erzeugen und Vermehren einer Lymphozytenpopulation, die wenigstens 90% CD4$^+$-Lymphozyten enthält, welches

(a) das Behandeln peripherer, mononukleärer Blutzellen mit 5-10 mM L-Leucyl-leucinmethylester oder 5-10 mM L-Leucinmethylester unter Abreichern von Monozyten und großer granulärer Lymphozyten und anschließend ohne positive Selektion oder Affinitätsentfernung von Nicht-CD4$^+$-Lymphozyten

(b) das ununterbrochene Kultivieren der peripheren, mononukleären Blutzellen in einem ein T-Zellenstimulans oder Interleukin-2 oder beides enthaltendem Kulturmedium bei 37°C während 4-21 Tagen

umfaßt.

**2.** Verfahren von Anspruch 1, bei welchem in Schritt (b) die Zellen in einem außer T-Zellstimulans Interleukin-2 enthaltendem Medium kultiviert werden.

**3.** Verfahren von Anspruch 1 oder 2, bei welchem die CD4$^+$-Zellen um etwa das 10- bis 1000fache vermehrt werden.

**4.** Verfahren von Anspruch 2, bei welchem die CD4$^+$-Zellen um wenigstens das 50fache vermehrt werden.

**5.** Verwendung einer durch das Verfahren von Anspruch 1 bis 4 erhaltenen Lymphozytenpopulation zur Herstellung eines Arzneimittels zur Behandlung einer durch einen Mangel an CD4$^+$-Lymphozyten gekennzeichneten Krankheit.

**6.** Verwendung gemäß Anspruch 5, wodurch die aus einem Virusinfizierten Patienten erhaltenen Zellen in Schritt (b) in Anwesenheit eines antiviralen Mittels in einer zum Hemmen der Virusreplikation und/oder -bindung wirksamen Menge kultiviert werden.

**7.** Verwendung gemäß Anspruch 6, wobei der Patient mit einem Human-Immunschwäche-Virus infiziert ist.

**8.** Verwendung gemäß Anspruch 7, wobei die Zellen in Schritt (b) in Anwesenheit von Azidothymidin kultiviert werden.

**9.** Verwendung gemäß Anspruch 7, wobei die Zellen in Schritt (b) in Anwesenheit von löslichem, rekombinantem CD4 kultiviert werden.

**10.** Verfahren zum Erzeugen und Vermehren einer an CD4$^+$ angereicherten, Tumor-infiltrierenden Lymphozytenpopulation, welches

(a) das Behandeln Tumor-infiltrierender Lymphozyten mit L-Leucyl-leucinmethylester unter Abreichern von Monozyten und großer granulärer Lymphozyten und anschließend ohne positive Selektion oder Affinitätsentfernung von Nicht-CD4$^+$-Lymphozyten

(b) das ununterbrochene Kultivieren der Tumor-infiltrierenden Leukozyten in einem ein T-Zellenstimulans oder Interleukin-2 oder beides enthaltendem Kulturmedium bei 37°C

umfaßt.

**11.** Verfahren von Anspruch 10, wobei die sich daraus ergebende Tumor-infiltrierende Leukozytenpopulation wenigstens 40% CD4$^+$-Lymphozyten enthält.

**12.** Verwendung der durch das Verfahren von Anspruch 11 erhaltenen Lymphozytenpopulation zur Herstellung eines Arzneimittels zum Behandeln von Krebs.

20

**13.** Zusammensetzung umfassend eine wäßrige Suspension menschlicher Lymphozyten, die durch die Verfahren von Anspruch 1-4 erhalten wurden, wovon mindestens 90% CD4$^+$-Lymphozyten sind, und Interleukin-2.

**14.** Zusammensetzung von Anspruch 13, worin die Lymphozyten periphere Blutlymphozyten sind.

**15.** Verfahren zum Herstellen eines Human-Immunschwäche-Virus, wobei periphere, mononukleäre Blutzellen aus einem mit dem Virus infizierten Patienten erhalten werden und mit dem Verfahren von Anspruch 1 behandelt werden, um eine an CD4$^+$-Lymphozyten angereicherte, Virus-infizierte Zellen enthaltende Zellpopulation zu liefern, und der Virus anschließend aus der Zellpopulation isoliert wird.

**16.** Verfahren zum Nachweis eines Human-Immunschwäche-Virus in peripheren, mononukleären Blutzellen, welches das Behandeln der Zellen mit dem Verfahren von Anspruch 1 unter Liefern einer an CD4$^+$-Lymphozyten angereicherten, virusinfizierte Zellen enthaltenden Zellpopulation und anschließend das Testen der Zellen auf die Anwesenheit des Virus, falls dieser vorhanden ist, umfaßt.

**17.** Verfahren von Anspruch 16, wobei die Zellen auf die Anwesenheit des Virus durch einen Hybridisierungstest oder Immuntest getestet werden.

**18.** Verfahren zum Herstellen autologer Zielzellen zur Verwendung in Tests zum Feststellen der Anwesenheit cytolytischer Effektorzellen in einer mit dem Human-Immunschwäche-Virus infizierten Person, welches das Erhalten peripherer, mononukleärer Blutzellen aus der Person, Behandeln der Zellen mit dem Verfahren von Anspruch 1 und anschließend das Hinzufügen von äußerem Hüllglykoprotein des Virus unter Erzeugen der Zielzellen umfaßt.

**Patentansprüche für folgenden Vertragsstaat : ES**

**1.** Verfahren zum Erzeugen und Vermehren einer Lymphozytenpopulation, die wenigstens 90% CD4$^+$-Lymphozyten enthält, welches
(a) das Behandeln peripherer, mononukleärer Blutzellen mit 5-10 mM L-Leucyl-leucinmethylester oder 5-10 mM L-Leucinmethylester unter Abreichern von Monozyten und großer granulärer Lymphozyten und anschließend ohne positive Selektion oder Affinitätsentfernung von Nicht-CD4$^+$-Lymphozyten
(b) das ununterbrochene Kultivieren der peripheren, mononukleären Blutzellen in einem ein T-Zellenstimulans oder Interleukin-2 oder beides enthaltendem Kulturmedium bei 37 °C während 4-21 Tagen
umfaßt.

**2.** Verfahren von Anspruch 1, bei welchem in Schritt (b) die Zellen in einem außer T-Zellstimulans Interleukin-2 enthaltendem Medium kultiviert werden.

**3.** Verfahren von Anspruch 1 oder 2, bei welchem die CD4$^+$-Zellen um etwa das 10- bis 1000fache vermehrt werden.

**4.** Verfahren von Anspruch 2, bei welchem die CD4$^+$-Zellen um wenigstens das 50fache vermehrt werden.

**5.** Verwendung einer durch das Verfahren von Anspruch 1 bis 4 erhaltenen Lymphozytenpopulation zur Herstellung eines Arzneimittels zur Behandlung einer durch einen Mangel an CD4$^+$-Lymphozyten gekennzeichneten Krankheit.

**6.** Verwendung gemäß Anspruch 5, wodurch die aus einem Virusinfizierten Patienten erhaltenen Zellen in Schritt (b) in Anwesenheit eines antiviralen Mittels in einer zum Hemmen der Virusreplikation und/oder -bindung wirksamen Menge kultiviert werden.

**7.** Verwendung gemäß Anspruch 6, wobei der Patient mit einem Human-Immunschwäche-Virus infiziert ist.

8. Verwendung gemäß Anspruch 7, wobei die Zellen in Schritt (b) in Anwesenheit von Azidothymidin kultiviert werden.

9. Verwendung gemäß Anspruch 7, wobei die Zellen in Schritt (b) in Anwesenheit von löslichem, rekombinantem CD4 kultiviert werden.

10. Verfahren zum Erzeugen und Vermehren einer an CD4$^+$ angereicherten, Tumor-infiltrierenden Lymphozytenpopulation, welches
(a) das Behandeln Tumor-infiltrierender Lymphozyten mit L-Leucyl-leucinmethylester unter Abreichern von Monozyten und großer granulärer Lymphozyten und anschließend ohne positive Selektion oder Affinitätsentfernung von Nicht-CD4$^+$-Lymphozyten
(b) das ununterbrochene Kultivieren der Tumor-infiltrierenden Leukozyten in einem ein T-Zellenstimulans oder Interleukin-2 oder beides enthaltendem Kulturmedium bei 37°C
umfaßt.

11. Verfahren von Anspruch 10, wobei die sich daraus ergebende Tumor-infiltrierende Leukozytenpopulation wenigstens 40% CD4$^+$-Lymphozyten enthält.

12. Verwendung der durch das Verfahren von Anspruch 11 erhaltenen Lymphozytenpopulation zur Herstellung eines Arzneimittels zum Behandeln von Krebs.

13. Verfahren zur Herstellung einer Zusammensetzung, die eine wäßrige Suspension menschlicher Lymphozyten, die durch die Verfahren von Anspruch 1-4 erhalten wurden, wovon mindestens 90% CD4$^+$-Lymphozyten sind, und Interleukin-2 umfaßt.

14. Verfahren von Anspruch 13, worin die Lymphozyten periphere Blutlymphozyten sind.

15. Verfahren zum Herstellen eines Human-Immunschwäche-Virus, wobei periphere, mononukleäre Blutzellen aus einem mit dem Virus infizierten Patienten erhalten werden und mit dem Verfahren von Anspruch 1 behandelt werden, um eine an CD4$^+$-Lymphozyten angereicherte, Virus-infizierte Zellen enthaltende Zellpopulation zu liefern, und der Virus anschließend aus der Zellpopulation isoliert wird.

16. Verfahren zum Nachweis eines Human-Immunschwäche-Virus in peripheren, mononukleären Blutzellen, welches das Behandeln der Zellen mit dem Verfahren von Anspruch 1 unter Liefern einer an CD4$^+$-Lymphozyten angereicherten, virusinfizierte Zellen enthaltenden Zellpopulation und anschließend das Testen der Zellen auf die Anwesenheit des Virus, falls dieser vorhanden ist, umfaßt.

17. Verfahren von Anspruch 16, wobei die Zellen auf die Anwesenheit des Virus durch einen Hybridisierungstest oder Immuntest getestet werden.

18. Verfahren zum Herstellen autologer Zielzellen zur Verwendung in Tests zum Feststellen der Anwesenheit cytolytischer Effektorzellen in einer mit dem Human-Immunschwäche-Virus infizierten Person, welches das Erhalten peripherer, mononukleärer Blutzellen aus der Person, Behandeln der Zellen mit dem Verfahren von Anspruch 1 und anschließend das Hinzufügen von äußerem Hüllglykoprotein des Virus unter Erzeugen der Zielzellen umfaßt.

**Revendications**

**Revendications pour les Etats contractants suivants : AT, BE, CH, DE, DK, FR, GB, IT, LI, LU, NL, SE**

1. Procédé pour générer et faire proliférer une population de lymphocytes contenant au moins 90% de lymphocytes CD4$^+$, qui comprend :
(a) le traitement des cellules mononucléaires du sang périphérique avec 5 à 10 mM d'ester méthylique de L-leucyl-leucine ou 5 à 10 mM d'ester méthylique de L-leucine pour les appauvrir en monocytes et en gros lymphocytes granulaires et ensuite, sans sélection positive ou élimination par affinité des lymphocytes non CD4$^+$,
(b) la culture de façon continue des cellules mononucléaires du sang périphérique dans un milieu de culture contenant un stimulant des cellules T ou de l'interleukine-2 ou les deux à 37C° pendant 4 à 21 jours.

22

2. Procédé selon la revendication 1, dans lequel, dans l'étape (b), les étapes sont cultivées dans un milieu contenant de l'interleukine-2 en plus du stimulant des cellules T.

3. Procédé selon les revendications 1 ou 2, dans lequel les cellules CD4$^+$ prolifèrent selon un facteur d'environ 10 à 1000 fois.

4. Procédé selon la revendication 2, dans lequel les cellules CD4$^+$ prolifèrent selon un facteur d'au moins 50 fois.

5. Utilisation d'une population de lymphocytes obtenue par le procédé selon les revendications 1 à 4 pour la production d'un médicament pour le traitement d'une maladie caractérisée par une déficience en lymphocytes CD4$^+$.

6. Utilisation selon la revendication 5, dans laquelle les cellules obtenues à partir des patients infectés par un virus sont cultivées dans l'étape (b) en présence d'un agent antiviral à une concentration efficace pour inhiber la réplication et/ou la liaison virale.

7. Utilisation selon la revendication 6, dans laquelle le patient est infecté par un virus de l'immunodéficience humaine.

8. Utilisation selon la revendication 7, dans laquelle les cellules sont cultivées dans l'étape (b) en présence d'azidothymidine.

9. Utilisation selon la revendication 7, dans laquelle les cellules sont cultivées dans l'étape (b) en présence de CD4 recombinant soluble.

10. Procédé pour générer et faire proliférer une population de lymphocytes infiltrant les tumeurs, enrichie en lymphocytes CD4$^+$, qui comprend :
    (a) le traitement des lymphocytes infiltrant les tumeurs avec l'ester méthylique de L-leucyl-leucine pour les appauvrir en monocytes et en gros lymphocytes granulaires et ensuite, sans sélection positive ou élimination par affinité des lymphocytes non CD4$^+$,
    (b) la culture de façon continue des lymphocytes infiltrant les tumeurs dans un milieu de culture contenant un stimulant des cellules T et de l'interleukine-2 à 37°C.

11. Procédé selon la revendication 10, dans lequel la population de lymphocytes infiltrant les tumeurs résultante contient au moins 40% de lymphocytes CD4$^+$.

12. Utilisation de la population de lymphocytes obtenue par le procédé selon le revendication 11, pour la production d'un médicament pour le traitement du cancer.

13. Composition comprenant une suspension aqueuse de lymphocytes humains obtenus par les procédés selon les revendications 1 à 4, dans lesquels au moins 90% sont des lymphocytes CD4+, et de l'interleukine-2.

14. Composition suivant la revendication 13, dans laquelle les lymphocytes sont des lymphocytes du sang périphérique.

15. Procédé pour produire un virus de l'immuno-déficience humaine dans lequel des cellules mononucléaires du sang périphérique sont obtenues chez un patient infecté par le virus et traitées par le procédé selon la revendication 1 pour fournir une population de cellules enrichie en lymphocytes CD4$^+$ contenant des cellules infectées par le virus, puis le virus est isolé à partir de la population de cellules.

16. Procédé pour la détection d'un virus de l'immunodéficience humaine dans les cellules mononucléaires du sang périphérique, qui comprend le traitement des cellules par le procédé selon la revendication 1 pour fournir une population de cellules enrichie en lymphocytes CD4$^+$ contenant des cellules infectées par le virus, s'il est présent, puis l'analyse des cellules pour déterminer la présence du virus.

**17.** Procédé selon la revendication 16, dans lequel les cellules sont testées pour déterminer la présence du virus par un test d'hybridation ou par un test immunologique.

**18.** Procédé pour produire des cellules cibles autologues utiles dans des tests pour déterminer la présence de cellules effectrices cytolytiques chez une personne infectée par un virus de l'immunodéficience humaine, qui comprend l'obtention de cellules mononucléaires du sang périphérique à partir de la personne, le traitement des cellules par le procédé selon la revendication 1, puis l'addition d'une glycoprotéine extérieure de l'enveloppe du virus pour produire des cellules cibles.

**Revendications pour l'Etat contractant suivant : ES**

**1.** Procédé pour générer et faire proliférer une population de lymphocytes contenant au moins 90% de lymphocytes CD4$^+$, qui comprend :

(a) le traitement des cellules mononucléaires du sang périphérique avec 5 à 10 mM d'ester méthylique de L-leucyl-leucine ou 5 à 10 mM d'ester méthylique de L-leucine pour les appauvrir en monocytes et en gros lymphocytes granulaires et ensuite, sans sélection positive ou élimination par affinité des lymphocytes non CD4$^+$,

(b) la culture de façon continue des cellules mononucléaires du sang périphérique dans un milieu de culture contenant un stimulant des cellules T ou de l'interleukine-2 ou les deux à 37C° pendant 4 à 21 jours.

**2.** Procédé selon la revendication 1, dans lequel, dans l'étape (b), les étapes sont cultivées dans un milieu contenant de l'interleukine-2 en plus du stimulant des cellules T.

**3.** Procédé selon les revendications 1 ou 2, dans lequel les cellules CD4$^+$ prolifèrent selon un facteur d'environ 10 à 1000 fois.

**4.** Procédé selon la revendication 2, dans lequel les cellules CD4$^+$ prolifèrent selon un facteur d'au moins 50 fois.

**5.** Utilisation d'une population de lymphocytes obtenue par le procédé selon les revendications 1 à 4 pour la production d'un médicament pour le traitement d'une maladie caractérisée par une déficience en lymphocytes CD4$^+$.

**6.** Utilisation selon la revendication 5, dans laquelle les cellules obtenues à partir des patients infectés par un virus sont cultivées dans l'étape (b) en présence d'un agent antiviral à une concentration efficace pour inhiber la réplication et/ou la liaison virale.

**7.** Utilisation selon la revendication 6, dans laquelle le patient est infecté par un virus de l'immunodéficience humaine.

**8.** Utilisation selon la revendication 7, dans laquelle les cellules sont cultivées dans l'étape (b) en présence d'azidothymidine.

**9.** Utilisation selon la revendication 7, dans laquelle les cellules sont cultivées dans l'étape (b) en présence de CD4 recombinant soluble.

**10.** Procédé pour générer et faire proliférer une population de lymphocytes infiltrant les tumeurs, enrichie en lymphocytes CD4$^+$, qui comprend:

(a) le traitement des lymphocytes infiltrant les tumeurs avec l'ester méthylique de L-leucyl-leucine pour les appauvrir en monocytes et en gros lymphocytes granulaires et ensuite, sans sélection positive ou élimination par affinité des lymphocytes non CD4$^+$,

(b) la culture de façon continue des lymphocytes infiltrant les tumeurs dans un milieu de culture contenant un stimulant des cellules T et de l'interleukine-2 à 37°C.

**11.** Procédé selon la revendication 10, dans lequel la population de lymphocytes infiltrant les tumeurs résultante contient au moins 40% de lymphocytes CD4$^+$.

**12.** Utilisation de la population de lymphocytes obtenue par le procédé selon le revendication 11, pour la production d'un médicament pour le traitement du cancer.

**13.** Procédé pour préparer une composition comprenant une suspension aqueuse de lymphocytes humains obtenus par les procédés selon les revendications 1 à 4, dans lesquels au moins 90% sont des lymphocytes CD4+, et de l'interleukine-2.

**14.** Procédé selon la revendication 13, dans lequel les lymphocytes sont des lymphocytes du sang périphérique.

**15.** Procédé pour produire un virus de l'immuno-déficience humaine dans lequel des cellules mononucléaires du sang périphérique sont obtenues chez un patient infecté par le virus et traitées par le procédé selon la revendication 1 pour fournir une population de cellules enrichie en lymphocytes CD4$^+$ contenant des cellules infectées par le virus, puis le virus est isolé à partir de la population de cellules.

**16.** Procédé pour la détection d'un virus de l'immunodéficience humaine dans les cellules mononucléaires du sang périphérique, qui comprend le traitement des cellules par le procédé selon la revendication 1 pour fournir une population de cellules enrichie en lymphocytes CD4$^+$ contenant des cellules infectées par le virus, s'il est présent, puis l'analyse des cellules pour déterminer la présence du virus.

**17.** Procédé selon la revendication 16, dans lequel les cellules sont testées pour déterminer la présence du virus par un test d'hybridation ou par un test immunologique.

**18.** Procédé pour produire des cellules cibles autologues utiles dans des tests pour déterminer la présence de cellules effectrices cytolytiques chez une personne infectée par un virus de l'immunodéficience humaine, qui comprend l'obtention de cellules mononucléaires du sang périphérique à partir de la personne, le traitement des cellules par le procédé selon la revendication 1, puis l'addition d'une glycoprotéine extérieure de l'enveloppe du virus pour produire des cellules cibles.